# EUROPEAN PATENT APPLICATION

(11) **EP 2 453 021 A1**
(43) Date of publication of application: **16.05.2012**
(21) Application number: 10190931.5
(22) Date of filing: 11.11.2010
(51) Int. Cl.: C12Q 1/04, C07D 209/10, C07D 209/30

(54) **Novel indicator compounds**

(71) Applicant: Biosynth AG, 9422 Thal (CH)
(72) Inventor: The designation of the inventor has not yet been filed
(74) Representative: Schmauder & Partner AG Patent- & Markenanwälte VSP

(57) **Abstract**

A novel class of chromogenic and fluorogenic indicators responding to external stimuli such as biochemical environment, temperature or photo irradiation comprises a plurality of compounds that are converted by an intramolecular aldol condensation to corresponding 10*H*-indolo[1,2-a]indole of (5*H*,7*H*)-indolo[1,2-a]quinoline signalophores. The indicators offer broad potential in a wide array of applications, particularly for biological systems.

## Description

### FIELD OF THE INVENTION

The invention discloses of a new class of chromogenic and fluorogenic indicators responding to external stimuli such as biochemical environment, temperature or photo irradiation. The novel indicators offer broad potential in a wide array of applications, particularly for biological systems.

### BACKGROUND OF THE INVENTION

Indicators are materials that produce a detectable signal in response to an external stimulus. Such stimuli typically include temperature, light (photo-labile or photochromic indicators), electric field (electrochromic indicators), pressure (piezoelectric indicators), ion concentration (e.g. pH indicators) and biochemical reactivity (e.g. enzyme indicators).

### PRIOR ART

Some known indicator compounds including some of their strengths and disadvantages are described below.

### Nitroreductase indicators

Nitroreductases are a family of enzymes capable of reducing nitrogen containing compounds including nitro compounds. They are found mainly in prokaryotic but also in some eukaryotic cells. *James et al.* have screened a variety of bacterial species and found nitroreductase activity to be present in all of them. They developed 7-nitrocoumarin derivatives as general indicators of microbial growth (James, Perry et al. 2001; James 2003). Because they are based on coumarin derivatives, assay products are diffusible and have to be detected by fluorescence. General growth indicators based on nitroreductase activity that yield colored or precipitating (colored and fluorescent) assay products would constitute a substantial progress.

James, A. L., J. D. Perry, et al. (2001). "Fluorogenic substrates for the detection of microbial nitroreductases." Letters in Applied Microbiology 33(6): 403-408.

James, A., D. Monget (2003). Fluorescent detection method for microorganisms based on nitrocoumarins. United States Patent 6555332,.

### Redox dyes

Redox dyes can be reduced by cellular dehydrogenases and reductases and are, therefore, used to indicate the viability of eukaryotic and prokaryotic cells. They can for example be used to asses the toxicity of chemical compounds, as a general growth indicator and for enumeration of live cells or cell colonies. Tetrazolium compounds and resazurin are most often employed for these purposes. Most tetrazolium dyes lead to colored insoluble formazans after reduction, although some soluble variants exist. The use and reliability of the fluorogenic tetrazolium CTC (5-cyano-2,3-ditolyl tetrazolium chloride) seems to be limited, because often an electron transfer mediator is needed for efficient reduction and fluorescence is not sufficient under several conditions. Resazurin is used as a soluble redox indicator and its reduction to resorufin can be followed both by colorimetric and also fluorometric methods. However, resorufin can be further reduced to the colorless, non-fluorescent dihydroresorufin. Redox dyes with stable fluorescent products (soluble and insoluble) would be a useful addition to the currently available dyes.

### β-Lactamase indicators:

β-Lactam antibiotics are an important class of antibiotics used against many infectious diseases. The broad use of β-lactam antibiotics caused β-lactamases, i.e. enzymes able to inactivate these antibiotics by hydrolysing their β-lactam ring, to spread through the bacterial populations. This made the early β-lactam antibiotics, like penicillin, useless and newer β-lactams, the cephalosporins, were synthesized that proved resistant to the normal β-lactamases. However, in the 1980s the so-called extended spectrum β-lactamases (ESBL) capable of hydrolysing the β-lactam ring of the third-generation cephalosporins emerged, drastically limiting the treatment options for many infectious diseases. To choose the right antibiotic therapy, the resistance pattern of the disease causing bacteria has to be determined as quickly as possible. Chromogenic and fluorogenic indicators specific for ESBLs are, therefore, extremely helpful tools for deciding on the most appropriate treatment of patients and for limiting the spread of ESBL expressing bacteria.

### DESCRIPTION OF THE INVENTION

It is an object of the present invention to provide a novel class of improved indicator compounds. In particular, these indicator compounds shall not have the disadvantages of the above mentioned known indicators.

A further object of the present invention is to provide an improved method for detecting an external stimulus in a region of interest.

Indicators are materials that produce a detectable signal in response to an external stimulus. Such stimuli typically include temperature, light (photo-labile or photochromic indicators), electric field (electrochromic indicators), pressure (piezoelectric indicators), ion concentration (e.g. pH indicators) and biochemical reactivity (e.g. enzyme indicators).

The mechanism of translation of stimulus into detectable signal is illustrated in Scheme I and typically involves the chemical removal or modification of a labile group (also denoted "LG") of the indicator in a process mediated by the experience of said stimulus.

**Scheme I**: Generic Concept and Terminology of Indicator Systems

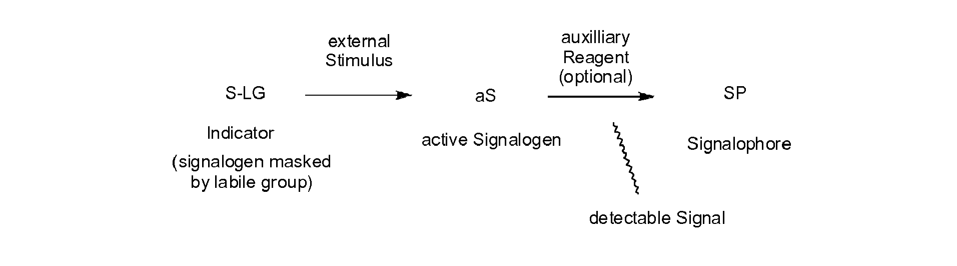

Removal or modification of the labile group most often yields an activated signalogen (also denoted "aS") which typically undergoes further transformation (with or without auxiliary reagents) to yield a signalophore (also denoted "SP"), the formation of which usually coincides with the occurrence of said detectable signal. Therefore, an indicator represents an activated signalogen that is masked, i.e. inactivated by a labile group. An indicator system comprises the elements of activated signalogen, labile group, auxiliary reagent (optional), detectable signal and suitable means for interrogation of detectable signal. The appearance of a detectable signal is a consequence of experiencing an external stimulus and hence allows ready detection and/or quantification of the external stimulus by interrogation with suitable instrumentation or the human eye.

According to one aspect of the invention, there is provided an indicator compound for detecting an external stimulus, the indicator compound being a compound of the general formula (Ia) 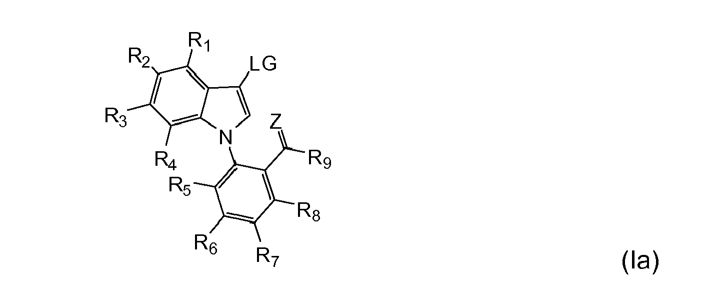 or (Ib) 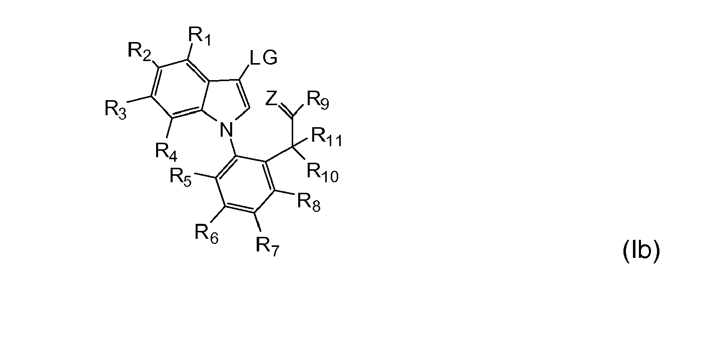 wherein
R1, R2, R3, R4, R5, R6, R7, R8, R9, R10 and R11 are independently selected from the group consisting of hydrogen; C1-4 alkyl; C1-4 alkoxy;
fused or linearly connected aryl; fused or linearly connected heteroaryl;
halogen; cyano; nitro; formyl and optionally substituted amino, carboxy, carbonyl, hydroxyl and sulfonyl;
Z is O, NH or S; and
LG is a labile group being susceptible to conversion into a π-donor containing moiety D by action of said external stimulus, so as to cause an intramolecular aldol condensation forming a compound of the general formula (IIa) 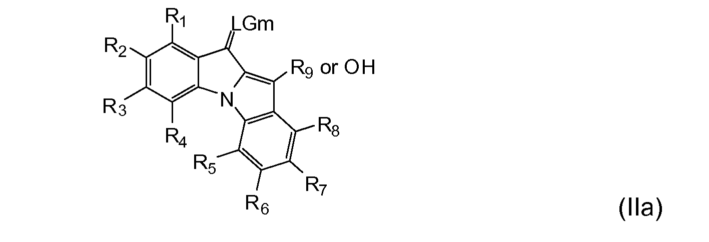 or (IIb) 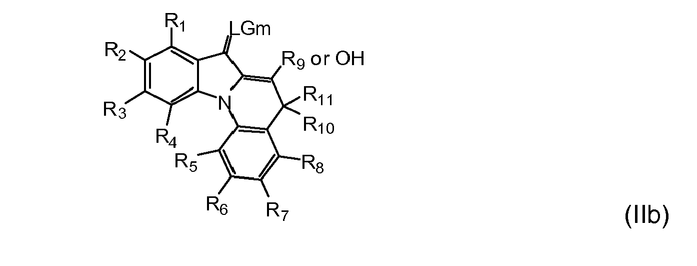 wherein LGm corresponds to said π-donor containing moiety D minus one electron.

The π-donor containing moiety D may simply consist of the π-donor or it may comprise the π-donor plus a further part forming a conjugated π-electron system, formed, for example a chain of C=C double bonds.

According to another aspect of the invention, there is provided a method for detecting an external stimulus in a region of interest, comprising the steps of:
- providing the region of interest with an indicator compound; and
- monitoring for a signal from a signalophore species formed as a consequence of said external stimulus;
characterized in that
said indicator compound is a compound of the general formula (Ia) or (Ib)
wherein R1, R2, R3, R4, R5, R6, R7, R8, R9, R10 and R11 are independently selected from the group consisting of hydrogen; C1-4 alkyl; C1-4 alkoxy; fused or linearly connected aryl; fused or linearly connected heteroaryl; halogen; cyano; nitro; formyl and optionally substituted amino, carboxy, carbonyl, hydroxyl and sulfonyl;
Z is O, NH or S; and
LG is a labile group being susceptible to conversion by action of said external stimulus, said conversion leading to formation of a signalogen species wherein said labile group LG is replaced by a π-donor containing moiety D, followed by an intramolecular aldol condensation forming said singalophore species, said signalophore species being a compound of the general formula (IIa) or (IIb), wherein LGm corresponds to said π-donor containing moiety D minus one electron.

It is well known in the art that ring-closure reactions proceed at particularly high rates if 5- or 6-membered rings are formed in the process. In the present invention, it was recognized that combining molecular moieties capable of acting as an aldol donor and acceptor, respectively, in such manner that the spatial arrangement of those moieties would strongly favor the event of an intramolecular aldol condensation. As a further essential point of the present invention, an untimely (i.e. too early or unselective) occurrence of the intramolecular aldol condensation is prevented by the masking effect of a suitable labile group conjugated to the aldol donor. Once the labile group is modified or removed by a certain external stimulus, the aldol condensation readily takes place and leads to formation of a signalophore.

The design of an intramolecular aldol indicator requires the aldol acceptor to be attached to the donor by means of a chemical structure linking the C-atom of the aldol acceptor (**Scheme II**). In the indicator compounds of the present invention, the linking may include one or two sequentially arranged atoms C₁ and C₂. In the former case a formal intramolecular aldol condensation yields a 5-membered ring whereas in the latter case it yields a 6-membered ring in the signalophore structure.

**Scheme II**: Basic Principle of Intramolecular Indox Aldol Indicators

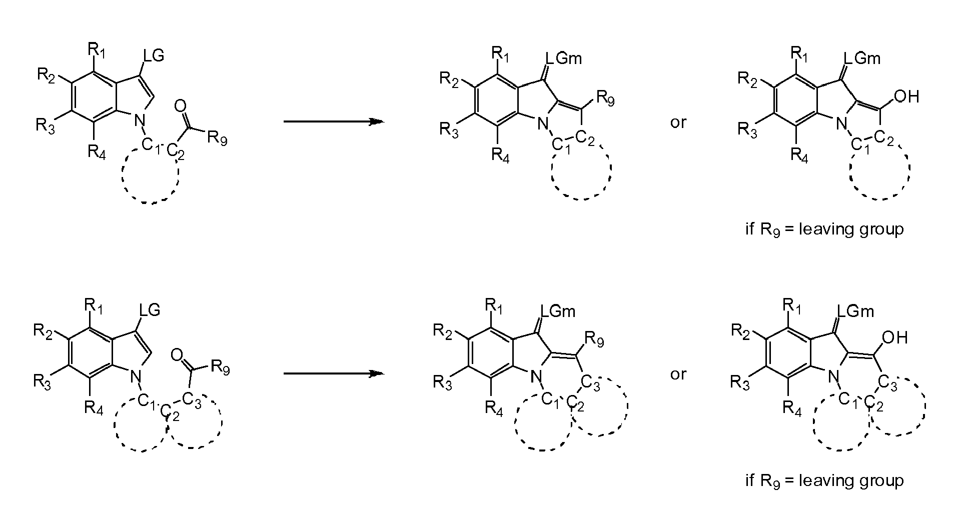

In the above Scheme II
- C₁, C₂, C₃ represent carbon atoms optionally substituted by hydrogen; C1-4 alkyl; fused or linearly connected aryl; fused or linearly connected heteroaryl; halogen; cyano; thio-cyano; nitro; nitroso; formyl; and optionally substituted amino, carboxy, carbonyl, hydroxyl, mercapto and sulfonyl
- R₁, R₂, R₃, R₄ are selected from the group consisting of hydrogen, C1-4 alkyl; fused or linearly connected aryl; fused or linearly connected heteroaryl; halogen; cyano; thiocyano; nitro; nitroso; formyl; and optionally substituted amino, carboxy, carbonyl, hydroxyl, mercapto and sulfonyl LG is a labile group as described in more detail below.

The aldol acceptor is preferably chosen as an aromatic carbonyl compound, (hence C₁ and C₂ represent members of an aromatic ring) such that an intramolecular aldol condensation would result in the formation of an extended conjugated system thereby providing the signalophore with desirable optical properties.

According to the present invention, the external stimulus transforms the LG into a π-donor containing moiety D.

It is generally understood that a substituent attached to a principal moiety will be considered a π-donor if it has an electron lone pair that may enter into conjugation with a π-system of the principal moiety, thereby increasing the π-system by at least one atom. A lone pair is a valence electron pair without bonding or sharing with other atoms. Lone pairs are found in the outermost electron shell of an atom, so they are a subset of a molecule's valence electrons. They can be identified by examining the outermost energy level of an atom - lone electron pairs consist of paired electrons as opposed to single electrons, which may appear if the atomic orbital is not full. Electron pairs are therefore considered lone pairs if two electrons are paired but are not used in chemical bonding. Thus, the number of lone electrons plus the number of bonding electrons equal the total number of valence electrons from a compound.

Therefore, in the present context the stimulus transforms the LG to a Lewis Base or increases the Lewis basicity of the LG.

The labile group may be selected from a large variety of chemical entities and will generally be selected according to the type of stimulus to be detected with the indicator compound.

In general, it will contain at least one functional group that provides the requested susceptibility to the stimulus of interest plus some group that will generate the requested π-donor. It is understood that these functionalities can be provided by one and the same structural unit.

**Table 1: List of functional groups in the labile groups (LG) and resulting π-donor containing moietys. Note that the actual π-donor might be separated from the 3 position of the indole ring by conjugated π-systems.**

| **Functional group in LG** | **π-Donor** |
|---|---|
| **Oxygen based** | Hydroxyl |
| Ethers, Alkyl, Allyl, Benzyl, Aryl, Propargyl, Vinyl | Hydroxyl |
| Esters, Alkyl, Allyl, Benzyl, Aryl, Propargyl, Vinyl | Hydroxyl |
| Inorganic Esters, Boric Acid Esters and Derivatives | Hydroxyl |
| Inorganic Esters, Mono-, Di- and Triphosphates | Hydroxyl |
| Inorganic Esters, Dithionates | Hydroxyl |
| Inorganic Esters, Dithionites | Hydroxyl |
| Inorganic Esters, Nitrates | Hydroxyl |
| Inorganic Esters, Nitrites | Hydroxyl |
| Inorganic Esters, Phosphites | Hydroxyl |
| Inorganic Esters, Polythionates | Hydroxyl |
| Inorganic Esters, Pyrophosphates | Hydroxyl |
| Inorganic Esters, Pyrosulfates | Hydroxyl |
| Inorganic Esters, Pyrosulfites | Hydroxyl |
| Inorganic Esters, Sulfates | Hydroxyl |
| Inorganic Esters, Sulfites | Hydroxyl |
| Inorganic Esters, Sulfoxylates | Hydroxyl |
| Inorganic Esters, Thiosulfates | Hydroxyl |
| Oxonium Salts | Hydroxyl |
| Peroxides | Hydroxyl |
| Cyanates | Hydroxyl |
| Enol Ethers | Hydroxyl |
| Hydroperoxides | Hydroxyl |
| Silyloxyanes, Derivatives of Silanol, Silandiol, Si-lantiol, Disilanhexol and Disiloxanol | Hydroxyl |
| Carbonates | Hydroxyl |
| Acetals | Hydroxyl |
| Oxime Ethers | Hydroxyl |
| | |
| **Nitrogen based** | Amines, Alkyl, Allyl, Benzyl, Aryl, Propargyl, Secondary, Ter-tiary |
| Ammonium salts | Amine |
| Iminium salts | Amine |
| Pyridinium salts | Amine |
| Nitro Compounds, Alkyl, Allyl, Benzyl, Aryl, Propargyl, Vinyl | Amine |
| Amidosulfonic Acids | Amine |
| Amine Oxides | Amine |
| Azides | Amine |
| Azo Compounds | Amine |
| Azoxy Compounds | Amine |
| Cyanamides | Amine |
| Diazo Compounds | Amine |
| Diazonium Salts | Amine |
| Hydroxyl Amine Derivatives | Amine |
| Isocyanates | Amine |
| Isonitriles | Amine |
| Isothiocyanates | Amine |
| Nitroso Compounds | Amine |
| Semicarbazid Derivatives and Isomers | Amine |
| Sulfinyl and Sulfonyl Amines | Amine |
| Triazenes | Amine |
| Triureas, Triuret | Amine |
| Ureas | Amine |
| Uronium Salts | Amine |
| Aminals | Amine |
| Hydrazones | Amine |
| Imines | Amine |
| Carboxylic Amides | Amine |
| Amidines | Amine |
| Formazanes | Amine |
| Hydrazides | Amine |
| Thioamides | Amine |
| | |
| **Sulfur based** | Sulfhydryl (Thiol / Mercaptane) Sulfides, Di, Tri and Poly |
| Bunte Salts | Sulfhydryl, Sulfide |
| Sulfenic Acids, Salts, Esters, Anhydrides, Halides, Amides, Imides and Hydrazids | Sulfhydryl |
| Sulfides, Di, Tri and Poly | Sulfhydryl |
| Sulfinic Acids, Salts, Esters, Anhydrides, Halides, Amides, Imides and Hydrazids | Sulfhydryl |
| Sulfones | Sulfhydryl, Sulfide |
| Sulfonic Acids, Salts, Esters, Anhydrides, Halides, Amides, Imides and Hydrazids | Sulfhydryl |
| Sulfonium Compounds | Sulfide |
| Sulfonyl Azides | Sulfhydryl |
| Sulfoxides | Sulfide |
| Thiocyanates | Sulfhydryl |
| Thioethers | Sulfhydryl |
| Thiohalides | Sulfhydryl |
| Mercaptals | Sulfhydryl |
| | |
| **Carbanion based** | Carbanion from Deprotonation Carbanion from Decarboxylation Ylides, N, S, P |
| Methanecarboxylic Acids and Esters, Alkyl, Allyl, Benzyl, Aryl | Carbanion from Deprotonation Carbanion from Decarboxylation |
| Methanecarboxylic Acids and Esters, Alpha-Beta-Unsaturated | Carbanion from Deprotonation Carbanion from Decarboxylation |
| Methanecarboxylic Acids and Esters, Cyano | Carbanion from Deprotonation Carbanion from Decarboxylation |
| Methanedicarboxylic Acids and Esters | Carbanion from Deprotonation Carbanion from Decarboxylation |
| Methanecarboxylic Acids and Esters, Hydroxy | Carbanion from Deprotonation Carbanion from Decarboxylation |
| Methanecarboxylic Acids and Esters, Oxo, Keto | Carbanion from Deprotonation Carbanion from Decarboxylation |
| Methanecarboxylic Acids and Esters, Unsaturated, Not Alpha-Beta | Carbanion from Deprotonation Carbanion from Decarboxylation |
| Ethyl Aldehydes, Alkyl, Aryl, Alpha-Beta-Unsaturated | Carbanion from Deprotonation |
| Ethyl Aldehydes, Oxo, Keto | Carbanion from Deprotonation |
| Ethyl Aldehydes, Poly | Carbanion from Deprotonation |
| Ethyl Aldehydes, Unsaturated, Not Alpha-Beta | Carbanion from Deprotonation |
| Ethyl Ketones, Alkyl, Aryl, Alpha-Beta-Unsaturated | Carbanion from Deprotonation |
| Ethyl Ketones, Cyano | Carbanion from Deprotonation |
| Ethyl Ketones, Halo | Carbanion from Deprotonation |
| Ethyl Ketones, Hydroxy | Carbanion from Deprotonation |
| Ethyl Ketones, Nitro | Carbanion from Deprotonation |
| Ethyl Ketones, Nitrogen Based | Carbanion from Deprotonation |
| Ethyl Ketones, Oxo, Keto | Carbanion from Deprotonation |
| Ethyl Ketones, Poly | Carbanion from Deprotonation |
| Ethyl Ketones, Propargyl | Carbanion from Deprotonation |
| Ethyl Ketones, Unsaturated, Not Alpha-Beta | Carbanion from Deprotonation |
| Ethyl Nitriles, Alkyl, Allyl, Benzyl, Aryl, Alkynyl | Carbanion from Deprotonation |
| Ethyl Nitriles, Alpha-Beta-Unsaturated | Carbanion from Deprotonation |
| Ethyl Nitriles, Cyano | Carbanion from Deprotonation |
| Ethyl Nitriles, Halo | Carbanion from Deprotonation |
| Ethyl Nitriles, Hydroxy | Carbanion from Deprotonation |
| Ethyl Nitriles, Nitrogen Based | Carbanion from Deprotonation |
| Ethyl Nitriles, Propargyl | Carbanion from Deprotonation |
| Ethyl Nitriles, Unsaturated, Not Alpha-Beta | Carbanion from Deprotonation |
| Phosphonium salts | Ylides |
| Ammonium Salts | Ylides |
| Sulfonium Salts | Ylides |

### EXAMPLES

Selected examples of the concept of the present invention are shown in Schemes III to VI below.

### Example 1

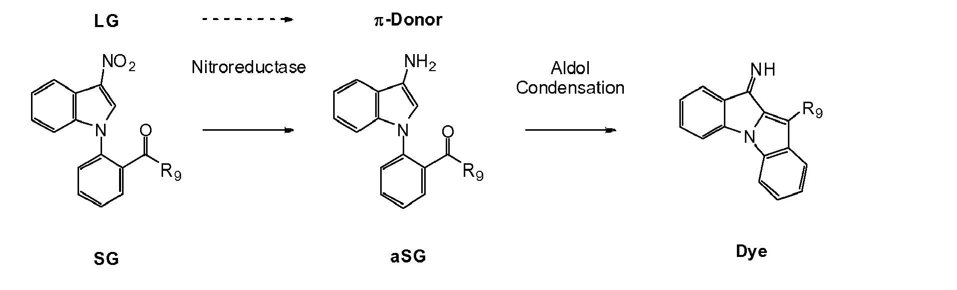

### Scheme III

In Scheme III, the LG is a nitro group (NO₂). Under the stimulus represented by the action of a nitroreductase, the LG is converted to an amine group (NH₂), which acts as a π-donor D. This effectively creates the prerequisites for enabling an intramolecular aldol condensation leading to formation of a well detectable indolo indole structure.

### Example 2

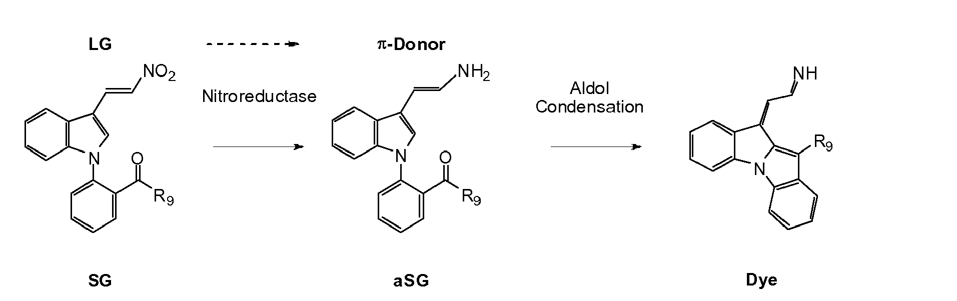

### Scheme IV

In Scheme IV, the LG is extended by a C=C double bond between the nitro group and the principal moiety. Accordingly, the conjugated system in the final dye structure is increased by one double bond in comparison with that of Scheme III. In the terminology used here, the π-donor is still NH₂ whereas the π-donor containing moiety is now -CH=CH-NH₂

### Example 3

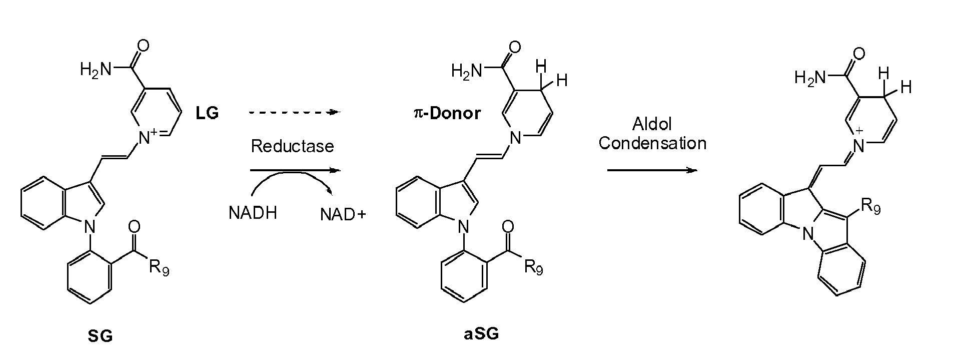

### Scheme V

In Scheme V, there is shown an indicator compound suitable for detection of a reductase stimulus.

### Example 4

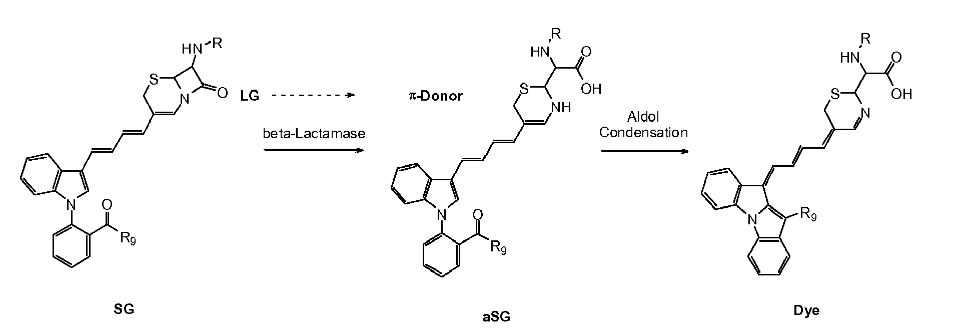

### Scheme VI

In Scheme VI, there is shown an indicator compound suitable for detection of a β-lactamase stimulus. Through the action of the β-lactamase an amide group is converted into an amino group (donor). The π-donor is separated from the 3 position of the indole ring by three conjugated C,C double bonds.

## Claims

1. An indicator compound for detecting an external stimulus, the indicator
compound being a compound of the general formula (Ia) 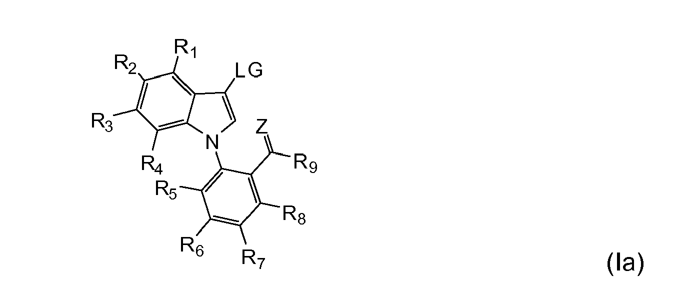 or (Ib) 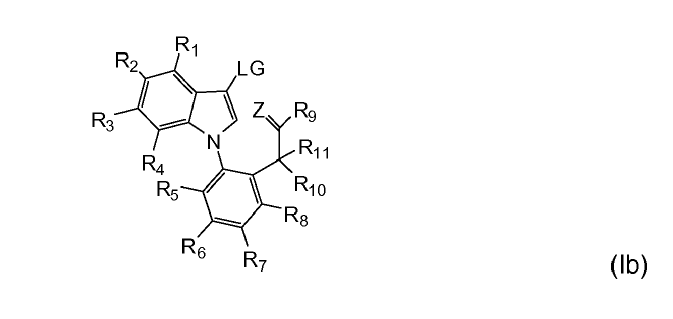 wherein
R1, R2, R3, R4, R5, R6, R7, R8, R9, R10 and R11 are independently selected from the group consisting of hydrogen; C1-4 alkyl; C1-4 alkoxy; fused or linearly connected aryl; fused or linearly connected heteroaryl; halogen; cyano; nitro; formyl and optionally substituted amino, carboxy, carbonyl, hydroxyl and sulfonyl;
Z is O, NH or S; and
LG is a labile group being susceptible to conversion into a π-donor containing moiety D by action of said external stimulus, so as to cause an intramolecular aldol condensation forming a compound of the general for- 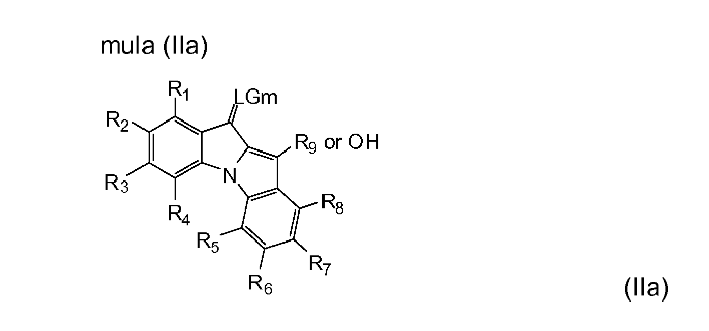 or (IIb) 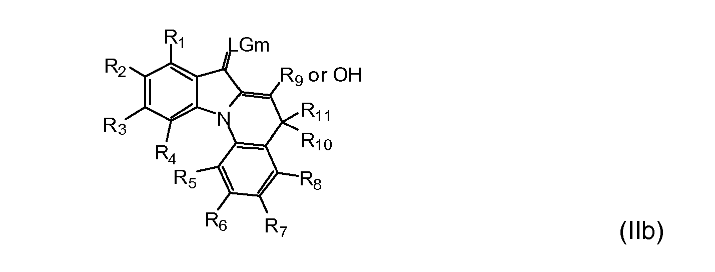 wherein LGm corresponds to said π-donor containing moiety D minus one electron.

2. The indicator compound according to claim 1, wherein the π-donor containing moiety D comprises a π-donor and a conjugated π-electron system.

3. The indicator compound according to claim 1, wherein LG is selected from the group according to **Table 1** in the description.

4. The indicator compound according to claim 1, with the exception of the compounds wherein LG is selected from the group consisting of beta-D-galactopyranoside, *tert*-butyldimethylsilyloxy (TBDMS), acetate, choline phosphate, alpha-D-glucopyranoside, beta-D-glucuronide sodium salt, N-acetyl-beta-D-galactosaminide and beta-D-glucopyranoside.

5. A method for detecting an external stimulus in a region of interest, comprising the steps of:
- providing the region of interest with an indicator compound; and
- monitoring for a signal from a signalophore species formed as a consequence of said external stimulus;
**characterized in that**
said indicator compound is a compound of the general formula (Ia) or (Ib) wherein R1, R2, R3, R4, R5, R6, R7, R8, R9, R10 and R11 are independently selected from the group consisting of hydrogen; C1-4 alkyl; C1-4 alkoxy; fused or linearly connected aryl; fused or linearly connected heteroaryl; halogen; cyano; nitro; formyl and optionally substituted amino, carboxy, carbonyl, hydroxyl and sulfonyl;
Z is O, NH or S; and
LG is a labile group being susceptible to conversion by action of said external stimulus, said conversion leading to formation of a signalogen species wherein said labile group LG is replaced by a π-donor containing moiety D, followed by an intramolecular aldol condensation forming said singalophore species, said signalophore species being a compound of the general formula (IIa) or (IIb), wherein LGm corresponds to said π-donor containing moiety D minus one electron.

6. The detection method according to claim 5, wherein the π-donor containing moiety D comprises a π-donor and a conjugated π-electron system.

7. The detection method according to claim 5, wherein LG is selected from the group according to Table 1 in the description.

8. The detection method according to claim 5, with the exception of the methods wherein LG is selected from the group consisting of beta-D-galactopyranoside, *tert*-butyldimethylsilyloxy (TBDMS), acetate, choline phosphate, alpha-D-glucopyranoside, beta-D-glucuronide sodium salt, *N-*acetyl-beta-D-galactosaminide and beta-D-glucopyranoside.
